# EUROPÄISCHE PATENTSCHRIFT

(11) **EP 1 501 806 B1**
(45) Veröffentlichungstag und Bekanntmachung des Hinweises auf die Patenterteilung: **29.03.2006**
(21) Anmeldenummer: 03722464.9
(22) Anmeldetag: 14.04.2003
(51) Int. Cl.: C07D 233/78, A61K 31/4166

(54) **ZUBEREITUNGEN ZUR TOPISCHEN APPLIKATION VON ANTIANDROGEN WIRKSAMEN SUBSTANZEN**
PREPARATIONS FOR THE TOPICAL APPLICATION OF ANTI-ANDROGENICALLY ACTIVE SUBSTANCES
PREPARATIONS POUR L'APPLICATION TOPIQUE DE SUBSTANCES ANTIANDROGENES

(30) Priorität: 27.04.2002 DE 10218963
(43) Veröffentlichungstag der Anmeldung: 02.02.2005
(73) Patentinhaber: Aventis Pharma S.A., 92160 Antony (FR)
(72) Erfinder: KRAEMER, Karl, Theodor, 63225 Langen (DE); NIETSCH, Karl-Heinz, 41470 Neuss (DE); POOTH, Rainer, 63303 Dreieich-Götzenhain (DE); MUENSTER, Uwe, La Jolla, CA 92037 (US); MEHNERT, Wolfgang, 13467 Berlin (DE); SCHAEFER-KORTING, Monika, 14195 Berlin (DE)
(74) Vertreter: Rousseau, Pierrick Edouard
(86) Internationale Anmeldenummer: PCT/EP2003/003837
(87) Internationale Veröffentlichungsnummer: WO 2003/093243

(56) Entgegenhaltungen:
- EP-A- 0 580 459
- AU-B- 726 572
- US-A- 5 411 981
- US-A- 6 162 444

## Beschreibung

Die androgenetische Alopezie ist die häufigste Form des Haarverlustes, der sowohl bei Männern als auch bei Frauen auftreten kann. Unter dem Begriff "androgenetische Alopezie" werden Haarmangelzustände verstanden, deren Ursache in der Regel eine genetisch determinierte Überempfindlichkeit der Haarwurzel auf 5α-Dihydrotestosteron (DHT) ist.

Ein typisches Beispiel einer androgenetischen Alopezie ist die gewöhnliche Glatze des Mannes. Eine androgenetische Alopezie kann jedoch auch bei Frauen im geschlechtsreifen Alter auftreten.

Die Behandlung des androgenetischen Haarausfalls setzt die frühzeitige Unterbrechung der pathogenetischen Vorgänge voraus, die zur Rückbildung des Haarfollikels führen. Um eine Normalisierung des Haarzyklus, d.h. eine Verlängerung der Wachstumsphase der Haare zu erreichen, muss die Stimulation der DHT-Rezeptoren in der dermalen Papille (Haarwurzel), d.h. der Wachstumszone des Haarschafts, reduziert werden. Dafür eignen sich prinzipiell die Blockade der Androgen (DHT-) Rezeptoren sowie die Verminderung der biologisch aktiven Androgenmenge in der dermalen Papille der Follikel. Wenn Endokrinopathien ausgeschlossen und Medikamente, die Testosteron oder andere androgen wirksame Substanzen enthalten, abgesetzt sind, ist die Hemmung der Androgenstimulation am Zielorgan notwendig. Zur Erreichung dieser Zielsetzung sind demnach theoretisch zwei Wege denkbar: Erstens, die Aktivitätshemmung der 5α-Reduktase und damit Minderung der Umwandlung von Testosteron in 5α-Dihydrotestosteron, beispielsweise durch Östrogen oder 5α-Reduktasehemmer, und zweitens eine Blockierung des Dihydrotestosteron-empfindlichen Rezeptorproteins, beispielsweise durch Antiandrogehe. Der zweite Weg lässt eine bessere Wirksamkeit erwarten, da keine Akkumulation des schwächer als DHT aber dennoch deutlich wirksamen Testosteron eintritt.

Da alle Therapiemaßnahmen bei der androgenetischen Alopezie sich gegen die Androgenwirkung richten, ist ihre systemische Anwendung bei gebärfähigen Frauen nur bei gleichzeitiger Kontrazeption möglich. Nach der Einführung der oralen Antikonzeptiva hat es sich gezeigt, dass je nachdem, ob man ein östrogenbetontes bzw. ein solches mit anti-androgener Partialwirkung oder ein Präparat mit androgener Restwirkung verabreicht, der Verlauf einer androgenetischen Alopezie und ihrer Begleitsymptome günstig oder ungünstig beeinflusst wird.

In Ermangelung einer anderen, stärker wirksamen, gefahrlosen Alternative werden zur topischen Behandlung der androgenetischen Alopezie bei Männern bislang östrogenhaltige Haarwässer verordnet. Bei Frauen wird diese Lokaltherapie als unterstützende Maßnahme empfohlen und das Hauptgewicht auf die systemische Behandlung mit einer Kombination aus einem Gestagen mit antiandrogener Partialwirkung und einem Östrogen gelegt. Bei einer androgenetischen Alopezie des Mannes kann darüber hinaus eine systemische Behandlung mit dem 5α-Reduktasehemmer Finasterid erfolgen, wobei allerdings der Erfolg begrenzt ist (Van Neste et al., Brit. J. Dermatol. 143, 804-10, 2000; McCleitan & Markham, Drugs, 57, 111-26, 1999).
Bei der Lokaltherapie werden alle Patienten angewiesen, den noch behaarten Bereich der Kopfhaut zu behandeln und nicht die bereits kahlen Bezirke. In vielen Fällen gelingt es mit Hilfe dieser Maßnahmen, die Schübe des Haarausfalls zu mildem oder zum Stillstand zu bringen. Eine Regeneration bereits atrophierter Haarfollikel (Glatze) ist nicht möglich.

Topisch wirksame Antiandrogene sind aus der französischen Patentschrift 2 693 461 und aus US 5,411,981 (4-[3-(4-Hydroxybutyl)-4,4-dimethyl-2,5-dioxo-1-imidazoli-dinyl]2-(trifluormethyl)-benzonitrile) bekannt, stehen aber derzeitig noch nicht zu Therapiezwecken zur allgemeinen Verfügung.

Beide Substanzklassen zeigen nach topischer Applikation eine hohe Bindungsaffinität zum Androgenrezeptor der Haarwurzel bei nahezu fehlender systemischer Aktivität.

Aufgrund der Substanz-inhärenten Teratogenität von Antiandrogenen mit Einfluss auf die Geschlechtsdifferenzierung im Spätstadium der Schwangerschaft sind die genannten Substanzen in Form von herkömmlichen wässrig / alkoholischen Haarwässem wegen des Auftretens von Substanzausfällungen an der Applikationsstelle nach Verdunsten des Lösungsmittels und dem damit verbundenen toxikologischen Risiko der Substanzübertragung auf Schwangere nicht verwendbar. Ferner ist durch herkömmliche Zubereitungen zum Auftragen auf die Kopfhaut die verzögerte Wirkstofffreisetzung über einen längeren Zeitraum zur Vermeidung von hohen systemischen Wirkstoffkonzentrationen und dem damit einhergehendem Auftreten von systemischen antiandrogenen Effekten nicht gewährleistet.

Um die in oben genannten Patenten antiandrogenen Wirkstoffe für eine sichere und wirksame Therapie zur Verfügung stellen zu können, war es daher erforderlich, Formulierungen zu finden, die die beschriebenen Nachteile von herkömmlichen Kopfhautbehandlungsmittein nicht aufweisen.

Die Aufgabe wird gelöst durch die erfindungsgemäßen Zubereitungen enthaltend ein oder mehrere Antiandrogenderivate der Formel I und Lipidnanopartikel bzw. eine Nanoemulsion. Die erfindungsgemäße Zubereitung ist vorteilhaft, weil die Lipidnanopartikel bzw. die Nanoemulsion bevorzugt zu den Haarfollikeln wandern und die Antiandrogenderivate der Formel I in hinreichend fester Verbindung zu den Lipiden (Lösung, stabile Adsorption) vorliegen und anschließend im Haarfollikel, durch Esterasen, in die wirksamen Antiandrogene gespalten werden. Ferner wird durch die erfindungsgemäßen Zubereitungen die unerwünschte Ausfällung der Antiandrogene an der Applikationsstelle verhindert. Eine Kontamination Dritter schließt auch die gute Mischbarkeit der Trägerlipide sowie der epidermalen Lipide aus. Die sehr enge Verbindung mit den körpereigenen Hautlipiden übertrifft die klassischer Topika (Creme, Salbe) deutlich.

Die Erfindung betrifft daher eine pharmazeutische Zubereitung, enthaltend mindestens eine Art von Lipidnanopartikeln und mindestens eine Verbindung der Formel I 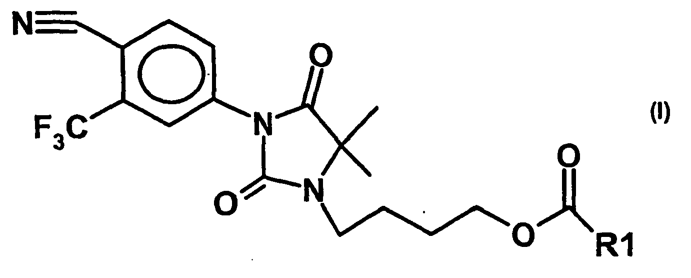 und /oder eine stereoisomere Form der Verbindung der Formel I
und/oder ein physiologisch verträgliches Salz der Verbindung der Formel I, worin R1 für -(C₅-C₁₇)-Alkyl oder -(C₅-C₁₇)-Alkenyl steht.

Ein weiterer Aspekt der Erfindung betrifft eine pharmazeutische Zubereitung, enthaltend Verbindungen der Formel I, worin
R1 für -(C₁₁-C₁₅)-Alkyl oder -(C₁₁-C₁₅)-Alkenyl steht.

Ein weiterer Aspekt der Erfindung betrifft eine pharmazeutische Zubereitung, enthaltend die Verbindung der Formel II.

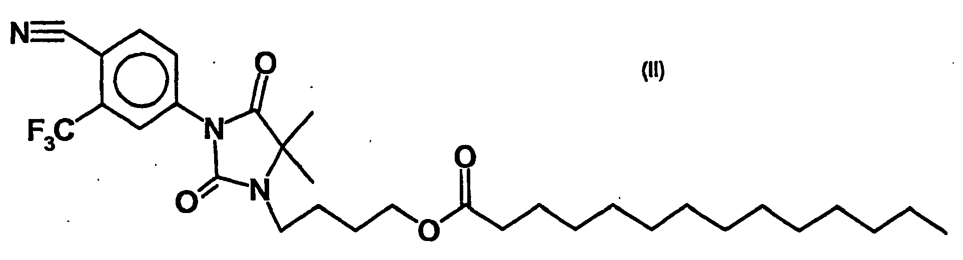

Die erfindungsgemäßen Zubereitungen zeichnen sich in erster Linie durch die Fähigkeit zu einer Anreicherung des Wirkstoffs im Haarfollikel aus. Als weitere Vorteile der erfindungsgemäßen Zubereitung sind eine gute Anhaftung an der Haut sowie ein Schutz des Wirkstoffs gegenüber Abbauprozessen in der Arzneiform zu nennen.

Dadurch wird sichergestellt, dass therapeutisch wirksame Antiändrogen-Konzentrationen an dem Zielorgan - der Haarwurzel - über einen längeren Zeitraum erreicht werden, ohne dass kurzfristig hohe Blutspiegelkonzentrationen auftreten, die naturgemäß zu einer systemischen Belastung des Patienten führen.

Ein weiterer Aspekt der Erfindung betrifft neue Verbindungen der Formel I 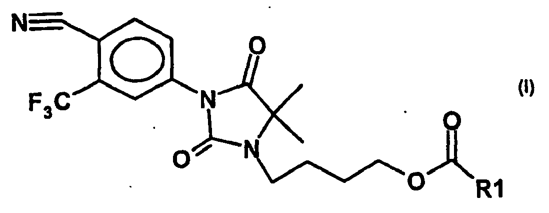 und /oder eine stereoisomere Form der Verbindung der Formel I und/oder ein physiologisch verträgliches Salz der Verbindung der Formel I, worin
R1 für -(C₅-C₁₇)-Alkyl oder -(C₅-C₁₇)-Alkenyl steht.

Ein weiterer Aspekt der Erfindung betrifft eine Verbindung der Formel I, worin R1 für -(C₁₁-C₁₅)- Alkyl oder -(C₁₁-C₁₅)- Alkenyl steht.

Ein weiterer Aspekt der Erfindung betrifft die Verbindung der Formel II.

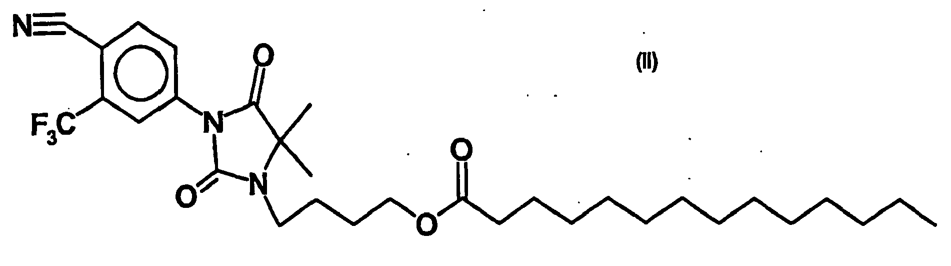

Unter dem Begriff "-(C₅-C₁₇)-Alkyl" werden Kohlenwasserstoffreste verstanden, deren Kohlenstoffkette geradkettig oder verzweigt ist und 5 bis 17 Kohlenstoffatome enthält, beispielsweise Pentyl, Iso-Pentyl, Neopentyl, Hexyl, 2,3-Dimethylbutyl, Neohexyl, Heptyl, Octanyl, Nonanyl, Decanyl, Dodecanyl, Pentadecanyl oder Heptadecanyl. Unter dem Begriff "(C₅-C₁₇)-Alkenyl" werden Kohlenwasserstoffreste wie die obengenannten (C₅-C₁₇)-Alkyl-Reste verstanden, deren Kohlenstoffkette geradkettig oder verzweigt ist und 5 bis 17 Kohlenstoffatome enthält und die je nach Kettenlänge zusätzlich 1, 2 oder 3 Doppelbindungen enthalten.

Die Antiandrogene sind bekannt und lassen sich durch literaturbekannte Verfahren herstellen (US 5,411,981).

Ein weiterer Aspekt der Erfindung betrifft ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel III 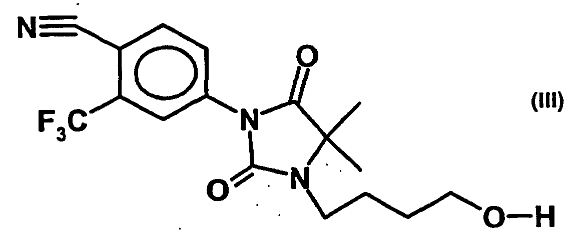 mit einer aktivierten Fettsäure der Formel IV 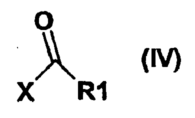 worin R1 wie in Formel I definiert ist und X ein Halogenrest ist, zu einer Verbindung der Formel I umsetzt, oder
b) eine nach Verfahren a) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler Verbindungen wie Aminosäuren, Trennung der somit erhaltenen Diastereomeren, und Abspaltung der chiralen Hilfsgruppen in die reinen Enantiomeren auftrennt, oder
c) die nach Verfahren a) hergestellte Verbindung der Formel I entweder in freier Form isoliert oder im Falle des Vorliegens von sauren oder basischen Gruppen in physiologisch verträgliche Salze überführt.

Die Umsetzungen erfolgen beispielsweise durch Reaktion eines Säurechlorids mit der alkoholischen Hydroxylgruppe in Gegenwart einer Base, beispielsweise Triethylamin, und einem organischen Lösungsmittel, beispielsweise Chloroform. Das Reaktionsprodukt wird chromatographisch gereinigt.

Im Verfahrensschritt b) wird die Verbindung der Formel I sofern sie in diastereoisomerer oder enantiomerer Form auftritt und bei der gewählten Synthese als deren Gemische anfällt, in die reinen Stereoisomeren getrennt, entweder durch Chromatographie an einem gegebenenfalls chiralen Trägermaterial, oder, sofern die racemischen Verbindungen der Formel I zur Salzbildung befähigt sind, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin. (+)- und (-)-Phenyl-ethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D-und L- Weinsäure, D-und L- Milchsäure sowie (+) und (-)-Mandelsäure in die reinen Enantiomeren überführen. Auch kann man chirale Verbindungen, die Alkohol- oder Aminfunktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter Methoden wieder abspaltet.

Saure oder basische Produkte der Verbindung der Formel I können in Form ihrer Salze oder in freier Form vorliegen. Bevorzugt sind pharmakologisch verträgliche Salze, z. B. Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschließlich deren stereoisomeren Formen, gemäß Verfahrensschritt c) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Omithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Ein weiterer Aspekt der Erfindung betrifft ein Verfahren zur Herstellung der erfindungsgemäßen Zubereitung, das dadurch gekennzeichnet ist, dass man die Verbindung der Formel I in einer heißen Lipid/Tensidlösung hochdruckhomogenisiert, wobei die Verbindung der Formel I eingeschlossen wird und anschließend abkühlt. Beim Abkühlen entsteht eine Dispersion fester Lipidpartikel, enthaltend die Verbindung der Formel I. Die Größe der Lipidpartikel beträgt weniger als 1 µm.

Ein weiterer Aspekt der Erfindung betrifft ein Verfahren zur Herstellung der erfindungsgemäßen Zubereitung, das dadurch gekennzeichnet ist, dass man die Verbindung der Formel I mit bei Raumtemperatur flüssigen Lipiden hochdruckhomogenisiert. Mit Raumtemperatur sind hierbei Temperaturen von 18 °C bis 25 °C gemeint. Einsetzbare Lipide sind beispielsweise Miglyol. Dieses Herstellverfahren führt zu sogenannten Nanoemulsionen, die sich durch den Einsatz bei Raumtemperatur flüssiger (z.B. Miglyol) anstelle von festen Lipiden von den festen Lipidnanopartikeln unterscheiden.

Im allgemeinen erfolgt die Herstellung der erfindungsgemäßen Zubereitungen in an sich bekannter Weise durch -Inkorporation der Verbindungen der Formel I-in die Partikel mittels Hochdruckhomogenisation.

Dazu werden beispielsweise in zwei Gefäßen ein Tensid (z.B. Poloxamer 188) und Wasser sowie die Verbindung der Formel I und das Lipid eingewogen. Beide Gefäße werden in einem Wasserbad auf die Temperatur erhitzt, bei der die heiße Homogenisation stattfinden soll. Diese Temperatur liegt in der Regel mindestens
10 °C über dem Schmelzpunkt des Lipids. Hierbei verflüssigt sich das Lipid. In der Schmelze des Lipids wird die Verbindung der Formel I gelöst. Nachdem die Lösungen annähernd die Temperatur des Wasserbades angenommen haben, wird die Tensidlösung zur Lipidlösung der Verbindung der Formel I gegeben.
Dieses Gemisch wird mit einem Rotor-Stator-Mischer (beispielsweise Ultra-Turrax) voremulgiert und anschließend unter Verwendung eines Hochdruckhomogenisators (z.B. EmulsiFex-B3, Avestin; LAB 40, Fa. APV-Gaulin) homogenisiert (z.B. 3 Zyklen bei 500 bar). Nach dem Herstellungsprozeß wird die gewonnene Lipid-Nanodispersion im Wasserbad bei beispielsweise 22 °C abgekühlt, wobei das Lipid unter Ausbildung von Lipid-Nanopartikeln auskristallisiert.

Die Lipid-Nanopartikel bestehen aus einer festen Lipidphase, die in einer emulgatorhaltigen wässrigen Phase dispergiert ist. Als Lipidphase werden physiologisch gut verträgliche Lipide eingesetzt, beispielsweise Glycerylbehenat oder Glycerylpalmitostearat und/oder Phosphatidylethanolamin, mit denen die Verbindung der Formel I nach Ausbildung der Lipidpartikel assoziiert vorliegt. Der Zusatz des Tensids dient der Stabilisierung der Lipid-Nanopartikel-Dispersion. Der mittlere Teilchendurchmesser von Lipid-Nanopartikeln liegt im Bereich von 50 nm bis 1000 nm, häufig im Bereich von 200 nm bis 400 nm.

Die erfindungsgemäßen Zubereitungen zeichnen sich in erster Linie durch einen gezielten Transport der Verbindung der Formel I in den Haarfollikel sowie durch eine verlangsamte Freisetzung der Verbindung der Formel I aus.

Vorzugsweise handelt es sich bei den pharmazeutischen Zubereitungen um flüssige Zubereitungen wie Haarwässer oder Haartonika, die als Hauptbestandteile Wasser und Lipide wie Precirol, Compritol, Monosteol, Imwitor (Glycerin-Mono-Stearat), Softisan (hydriertes Palmenöl), Miglyol (Caprinsäure-Caprylsäure-Triglycerid) oder Phosphatidylethanolamin, sowie Tenside (z.B. Poloxamer), aber auch wässrigen (C₁-C₆)-Alkohol, wie beispielsweise Ethanol, Propanol, Butanol, Pentanol, Hexanol oder Isopropanol enthalten können, ferner um Lotionen oder um halbfeste Zubereitungen wie Emulsionen, Cremes, Gele oder Salben. Die Zubereitungen können auch in Aerosolform vorliegen. Als Zusatzstoffe können die erfindungsgemäßen Zubereitungen auch mindestens eine durchblutungsfördemde Verbindung enthalten wie Dihydralazin, Diisopropylamin, Aminexil, Diazoxid oder Calciumantagonisten wie Nifedipin, Nicardipin, Verapamil, Diltiazem, Nisoldipin, Nitrendipin, Nivaldipin, Isradipin, Felodipin, Nimodipin, Gallopamil, Fendilin, Flunarizin, Amlodipin, Diperdipin, Fluspirilen, Primozid, Fantofaron, Nicergolin oder Cyclandelat, 6-Amino-4-piperidino-1,2-dihydro-1-hydroxy-2-iminopyrimidin (Minoxidil), Angiotensin-Converting-Enzym-Hemmer wie Quinapril, Lisinopril, Benzazepril, Captopril, Ramipril, Fosinopril, Cifazapril oder Trandolapril, Methylxanthinverbindungen wie Pentoxifyllin, Propentofyllin, Torbafyllin oder deren Mischung.

Geeignete Zusatzstoffe sind auch mindestens ein Natriumkanalöffnerwie 1-Cyan-2-(1,1-dimethyl-propyl)-3-(3-pyridyl)-guanidin oder 5-alpha-Reduktasehemmer wie N-tertiär-Butyl-3-oxo-4aza-5α-androst-1-en-17β-carboxamid. Weitere geeignete Zusatzstoffe sind auch mindestens eine haarwachstumsfördemde Verbindung wie ein inneres Salz von 2,4-Diamino-6-alkoxy-3-sulfoxypyrimidinhydroxid mit 1 bis 6 Kohlenstoffatomen im Alkoxyrest wie beschrieben in EP 0 427 625; z.B. das innere Salz von 2,4-Diamino-6-butoxy-3-sulfoxypyrimidinhydroxid, oder Pyridin-1-oxid-derivate wie beschrieben in WO 92 21317; z.B. 2,6-Diamino-4-piperidinopyridin, oder 2,6-Diamino-1,3,5-triazin-derivate wie beschrieben in WO 91 19701; z.B. 2,6-Diamino-4-butoxy-1,3,5-triazin-1-oxid. Ferner sind auch Mischungen der genannten Zusatzstoffe geeignet.

Als weitere Zusatzstoffe können die erfindungsgemäßen Zubereitungen die in der Kosmetik üblichen haar- und kopfhautpflegenden Substanzen und medizinische Wirkstoffe enthalten wie beispielsweise Antischuppenmittel, antiseborrhoeisch wirksame Präparate, Stoffe mit keratolytischer und keratoplastischer Wirkung wie Salicylsäure, Allantoin, Schwefelpräparate, Harnstoff, Ceramide, Antimikrobica, Vitamine, Pflanzen- oder Organextrakte, Hormone, Corticoide, Hyperämica, wie Nikotinsäure und deren Derivate, organische Säuren wie Zitronensäure, Orotsäure, Liponsäure, Aminosäuren, polyoxäthylierte Fettalkohole, Fettsäuren, Sorbitanfettsäureester, Alkylphosphate und Öle, z.B. Fettsäureester, sowie ferner Konservierungsmittel, Farbstoffe und Parfümöle. Wesentlich ist, dass die Zusatzstoffe mit antiandrogenen Substanzen kompatibel sind und deren Haarwuchswirkung nicht inhibieren. Darüber hinaus dürfen sie auch die systemische Aufnahme des Antiandrogens nicht fördern.

Mit den erfindungsgemäßen Zubereitungen lässt sich die Behandlung der androgenetischen Alopezie sicher und wirksam durchführen. Im Hinblick auf die bisherigen schlechten Therapieerfolge ist dies ein überaus wichtiger Befund.

Die erfindungsgemäßen Zubereitungen sind auch zur Behandlung des Hirsutismus, das heißt zur Vermeidung von unerwünschter Behaarung und zur Behandlung der Seborrhö und Akne geeignet.

In den erfindungsgemäßen Zubereitungen ist der Wirkstoff im allgemeinen in einer Menge von 0,01 Gewichtsprozent bis 10 Gewichtsprozent, vorzugsweise 0,1 bis 5 Gewichtsprozent enthalten.

Die Erfindung betrifft ferner den Einsatz der erfindungsgemäßen Zubereitungen in der Kosmetik.

### Beispiel 1

### Herstellung der Verbindung der Formel II

300 mg 4-[3-(4-Hydroxybutyl)-4,4-dimethyl-2,5-dioxo-1-imidazolidinyl]-2-(trifluormethyl)-benzonitril, im folgenden Verbindung 1, (8,13 × 10⁻⁴ mol) wurden mit 400 mg Myristinsäurechlorid (1,62 × 10⁻³ mol) in Gegenwart von 0,5 ml Triethylamin in 10 ml absolutem Chloroform 24 Stunden (h) unter Rühren umgesetzt. Nach Ablauf der Reaktion wurde per DC - Kontrolle (Kieselgel-Platte, Fließmittel Ethylacetat) die Bildung eines lipophilen Produktes beobachtet. Die quantitative Abtrennung des mutmaßlichen Esters erfolgte mit einem Chromatotron der Firma Harrison Research (Palo alto, USA) mit Methylenchlorid als Elutionsmittel. Die organische Lösung des abgetrennten Reaktionsproduktes wurde eingedampft, mit Wasser aus Methanol/Chloroform umkristallisiert und getrocknet. Die Identifizierung der Verbindung der Formel II erfolgte anhand von ¹H NMR (400 MHz) Spektroskopie, Massenspektroskopie und C-H-N-Analyse sowie ¹³C-NMR sowie H-H und C-H Cosy Spektren:
¹H-NMR (CDCl₃; 400,132 MHz, ppm): 0,88 (m, 3 H, CH₃); 1,25 (m, 20 H, CH₂); 1,54 (s, 6 H, CH₃); 1,69-1,81 (m, 6 H, CH₂); 2,30 (m, 2 H, CH₂); 3,39 (m, 2 H, CH₂); 4,13 (m, 2 H, CH₂); 7,91 (d, 1 H, ar); 8,01 (d, 1 H, ar); 8,16 (s, 1 H, ar).
¹³C-NMR (CDCl₃; 100,625 MHz): distinkte Signale bei ppm: 14,12; 22,69; 23,51; 25,0; 26,15;26,30; 29,18; 29,28; 29,36; 29,48; 29,61; 29,65; 29,68; 31,93; 34,32; 40,0; 61,87; 63,34; 1208,25; 115,02; 122,89; 122,94; 122,97; 123,04; 123,35; 127,85; 135,27; 136,50; 152,85; 173,67; 174,56.
Der Schmelzpunkt der Verbindung der Formel II liegt bei 70,7 °C bis 72,4 °C.
Die Ausbeute betrug 260 mg (4,49 × 10⁻⁴ mol). Dies entspricht einer Ausbeute von 55,2 %.
Massenspektrum MS: 579,7

| Molekulare Zusammensetzung: | | | | | | | | | |
|---|---|---|---|---|---|---|---|---|---|
| C | 64,23 %; | H | 7,65 %; | F | 9,83 %; | N | 7,25 %; | O | 11,04 % |

### CHN-Analyse:

| Atom | Theoret. Wert | 1. Messung | 2. Messung |
|---|---|---|---|
| C | 64,23 | 63,97 | 63,86 |
| H | 7,651 | 7,706 | 7,945 |
| N | 7,249 | 6,942 | 7,029 |

### Beispiel 2

Die erfindungsgemäße Zubereitung weist beispielsweise folgende Zusammensetzung (% Gewicht) auf:

| a) | |
|---|---|
| Verbindung der Formel II | 0,1-1% |
| Compritol (15 % Mono-, 50 % Di- und 35 % Triglycerid der Behensäure) | 5 % |
| Poloxamer 188 (Polyoxyethylen-Polyoxypropylen Polymer) | 1,25 % |

| b) | |
|---|---|
| Verbindung der Formel II | 0,1-1% |
| Precirol (Glycerin-Palmitat-Stearat) | 5 % |
| Poloxamer 188 | 1,25 % |

| c) | |
|---|---|
| Verbindung der Formel II | 0,1 -1% |
| Monosteol (Propylenglycol-Palmitat-Stearat) | 5 % |
| Poloxamer 188 | 1,25 % |

### Beispiel 3

### Herstellung der Zubereitung

In zwei Gefäßen wurden 0,05 g Poloxamer 188 und 3,746 ml Wasser sowie 0,004 g der Verbindung gemäß Beispiel 1, im folgenden Verbindung 2, und 0,2 g des Lipids (beispielsweise Precirol) eingewogen. Beide Gefäße wurden in einem Wasserbad auf die Temperatur von 80 °C erhitzt. Hierbei verflüssigt sich das Lipid. In der Schmelze des Lipids wurde die Verbindung 2 gelöst. Nachdem die Lösungen annähernd die Temperatur des Wasserbades angenommen hatten, wurde die Tensidlösung zur Lipidlösung bzw. Lipiddispersion der Verbindung der Formel I gegeben.
Dieses Gemisch wurde mit einem Rotor-Stator-Mischer (Ultra-Turrax) 8000 UPM, 10 sec voremulgiert und anschließend unter Verwendung eines Hochdruckhomogenisators (EmulsiFlex-B3, Avestin) mit 3 Zyklen bei 500 bar homogenisiert. Nach dem Herstellungsprozeß wurde die gewonnene Lipid-Nanodispersion im Wasserbad auf eine Temperatur von 22 °C abgekühlt, wobei das Lipid unter Ausbildung von Lipid-Nanopartikeln auskristallisiert. Die Ausbeute an kristallinem Lipid betrug 98,3%.

Die hergestellten Nanopartikel und die Nanoemulsion wurden wie folgt physikalisch charakterisiert:

**Tabelle 1: Laserdiffraktometrie (LD)**

| | | Tag 3 (4) | | Tag 16 | | Tag 44 | |
|---|---|---|---|---|---|---|---|
| Lipid | | LD 50 % (µm) | LD 95 % (µm) | LD 50 % (µm) | LD 95 % (µm) | LD 50 % (µm) | LD 95 % (µm) |
| Compritol | mit Verbindung 2 | 0,214 | 0,563 | 0,211 | 0,640 | n. d. | n. d. |
| | ohne Verbindung 2 | 0,249 | 0,586 | 0,260 | 0,615 | n. d. | n. d. |
| Precirol | mit Verbindung 2 | 0,199 | 1,963 | 0,181 | 1,544. | 0,108 | 0,295 |
| | ohne Verbindung 2 | 0,151 | 0,843 | 0,185 | 1,877 | 0,089 | 0,249 |
| Monosteol | mit Verbindung 2 | 36,62 | 74,01 | 20,72 | 46,25 | n. d. | n. d. |
| | ohne Verbindung 2 | 19,47 | 112,5 | 16,65 | 51,01 | n. d. | n. d. |
| Nanoemulsion (Miglyol) | | 0,171 | 0,920 | 0,149 | 0,149 | n. d. | n. d. |

| | | | | | | | |
|---|---|---|---|---|---|---|---|
| LD 50% (LD 95%): 50% (bzw. 95%) der Partikel sind kleiner als der angegebene Durchmesser | | | | | | | |
| (nach: Mehnert & Mäder, Adv. Drug Delivery Rev. 47, 165-196, 2001) | | | | | | | |

**Tabelle 2: Photonencorrelationsspektroskopie (PCS)**

| | | Mittlerer Durchmesser (µm) | | |
|---|---|---|---|---|
| Lipid | SLN | Tag 3 (4) | Tag 16 | Tag 44 |
| Compritol | mit Verbindung 2 | 0,255 | 0,214 | n.d. |
| | ohne Verbindung 2 | 0,259 | n.d. | n.d. |
| Precirol | mit Verbindung 2 | 0,214 | n.d. | 0,211. |
| | ohne Verbindung 2 | 0,224 | n.d. | n.d. |
| Monosteol | mit Verbindung 2 | 0,266 | n.d. | n.d. |
| | ohne Verbindung 2 | 0,261 | n.d. | n.d. |

| | | | | |
|---|---|---|---|---|
| n.d. bedeutet nicht bestimmt | | | | |
| (nach: Mehnert & Mäder, Adv. Drug Delivery Rev. 47, 165-196, 2001) | | | | |

Die Ergebnisse zeigen eine weithin stabile Partikelgröße bei der Lagerung. Zudem beeinflusst die Inkorporation des Wirkstoffs die Stabilität der Zubereitung nicht negativ. Ausgeprägte Schmelzpeaks bei der Untersuchung mittels Differentialkalorimetrie belegen zudem, dass es sich um feste Partikel handelt. Mikroskopische Untersuchungen ergaben keine Anzeichen einer Auskristallisation der Verbindung 2.

### Beispiel 4

Untersuchungen zur Rezeptoraffinität der Verbindungen 1 und 2 erfolgten an 29+/GR+ Zellen, die den Androgenrezeptor exprimieren (List et al.; Exp. Cell Res. 250; 414-422; 1999). Die Affinität wurde vergleichend zu Dihydrotestosteron (DHT) bestimmt. Als EC₅₀₋Werte wurden erhalten DHT 0,27 nM; Verbindung 1 6,7 nM und Verbindung 2 11045 nM. Wahrscheinlich beruht die Bindung von Verbindung 2 nicht auf die Esterfunktion, sondern auf einer enzymatischen oder spontanen Esterhydrolyse. Verbindung 2 ist demnach ein Prodrug von Verbindung 1.

Die Freisetzung der aktiven Verbindung 1 aus den Lipidpartikeln, worin sie als Verbindung 2 vorliegt, wurde durch Untersuchungen an Hautzellkulturen belegt. Monolayerkulturen juveniler Vorhautkeratinozyten und -fibroblasten sowie von Zellen der dermalen Papille okzipitaler Kopfhaut wurden für 24 h mit der Verbindung 2 in 10⁻⁵ M Konzentration unter Standardbedingungen (5% CO₂, 37° C) inkubiert. Anschließend wurde das Zellkulturmedium mit Chloroform extrahiert. Die organische Phase wurde eingedampft und der mit Acetonitril aufgenommene Rückstand wurde HPLC-analytisch auf seinen Gehalt an den Verbindungen 1 und 2 untersucht.

**Tabelle 3: Hydrolyse von Verbindung 2 in kultivierten human Hautzellen (DP, Dermale Papille; FB, Fibroblasten; KC, Keratinozyten). Die Zellen wurden 24 h mit Verbindung 2 unter Standardbedingungen (5 % CO₂, 37 °C) kultiviert. Extrakte der Medien wurden mittels HPLC analysiert '(n=3).**

| | Verbindung 1- Bildung | |
|---|---|---|
| Stamm | pmol/µg Protein | % pro Ansatz |
| DP 03/99 | 177,9 ± 23,4 | 49,6 ± 6,5 |
| FB x2712 | 166,7 ± 24,7 | 34,2 ± 5,1 |
| FB x1412 | 158,9 ± 17,0 | 56,1 ± 6.0 |
| FB x1 | 81.4 ± 12,7 | 57,0 ± 8.9 |
| KC x608 | 21,6 ± 0,8 | 21,6 ± 0,8 |
| KC x709 | 20,3 ± 1,3 | 25,4 ± 1,7 |

Daraus folgt, dass es in Zellen der Haut zu einer signifikanten Umwandlung der Verbindung 2 in die Verbindung 1 kommt. Dies gilt vor allen Dingen auch für die Zellen der dermalen Papille, die das Target für Antiandrogene wie die Verbindung 1 darstellen.

## Patentansprüche

1. Verbindung der Formel I 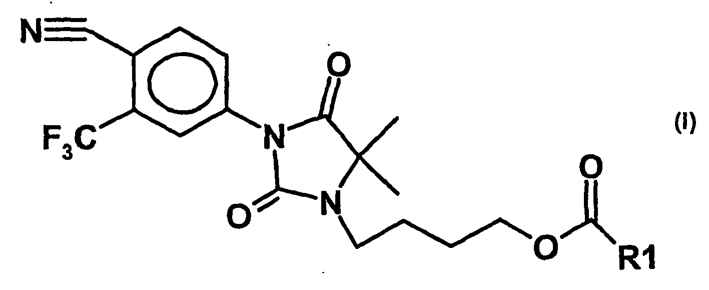 und /oder eine stereoisomere Form der Verbindung der Formel I und/oder ein physiologisch verträgliches Salz der Verbindung der Formel I, worin
R1 für -(C₅-C₁₇-Alkyl oder -(C₅-C₁₇)-Alkenyl steht.

2. Verbindung der Formel I gemäß Anspruch 1, **dadurch gekennzeichnet, dass**
R1 für -(C₁₁-C₁₅)-Alkyl oder -(C₁₁-C₁₅)-Alkenyl steht.

3. Verbindung der Formel I gemäß der Ansprüche 1 oder 2, **dadurch gekennzeichnet, dass** sie die Verbindung der Formel II 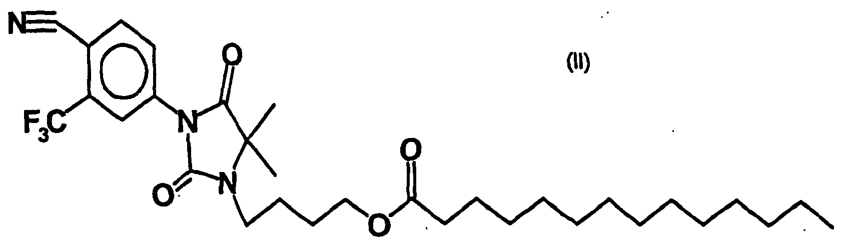 ist.

4. Pharmazeutische Zubereitung, enthaltend mindestens ein Lipidnanopartikel und mindestens eine Verbindung der Formel I gemäß Anspruch 1 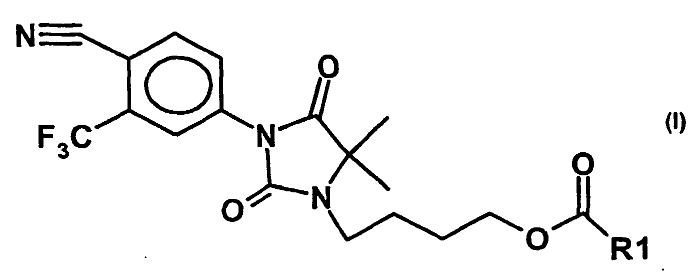 und /oder eine stereoisomere Form der Verbindung der Formel I
und/oder ein physiologisch verträgliches Salz der Verbindung der Formel 1, worin
R1 für -(C₅-C₁₇)-Alkyl oder -(C₅-C₁₇)-Alkenyl steht.

5. Pharmazeutische Zubereitung gemäß Anspruch 4, **dadurch gekennzeichnet, dass** sie eine Verbindung der Formel I enthält, worin
R1 für -(C₁₁-C₁₅)-Alkyl oder -(C₁₁-C₁₅)-Alkenyl steht.

6. Pharmazeutische Zubereitung gemäß der Ansprüche 4 oder 5, **dadurch gekennzeichnet, dass** sie eine Verbindung der Formel II 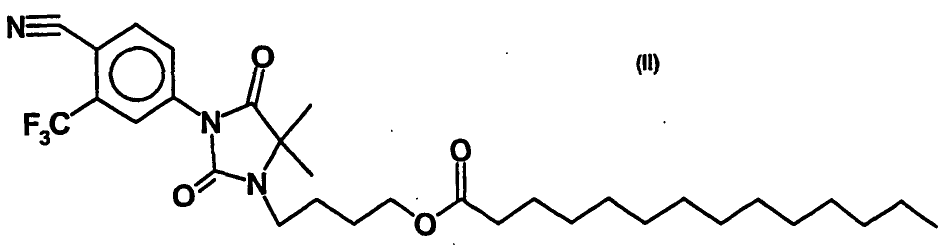 enthält.

7. Verfahren zur Herstellung der Verbindung der Formel I gemäß einem oder mehreren der Ansprüche 1 bis 3, **dadurch gekennzeichnet ist, dass** man
a) eine Verbindung der Formel III 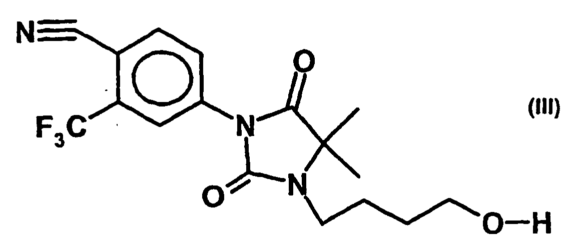 mit einer aktivierten Fettsäure der Formel IV 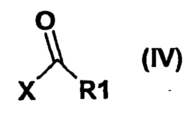 worin R1 wie in Formel I definiert ist und X ein Halogenrest ist, zu einer Verbindung der Formel I umsetzt, oder
b) eine nach Verfahren a) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler Verbindungen wie Aminosäuren, Trennung der somit erhaltenen Diastereomeren, und Abspaltung der chiralen Hilfsgruppen in die reinen Enantiomeren auftrennt, oder
c) die nach Verfahren a) hergestellte Verbindung der Formel I entweder in freier Form isoliert oder im Falle des Vorliegens von sauren oder basischen Gruppen in physiologisch verträgliche Salze überführt.

8. Arzneimittel, **gekennzeichnet durch** einen wirksamen Gehalt an mindestens einer Verbindung der Formel I gemäß einem oder mehreren der Ansprüche 1 bis 3 zusammen mit einem pharmazeutisch geeigneten und physiologisch verträglichen Trägerstoff, Zusatzstoff und/oder anderen Wirk- und Hilfsstoffen.

9. Verfahren zur Herstellung der Pharmazeutischen Zubereitung gemäß einem oder mehreren der Ansprüche 4 bis 6, **dadurch gekennzeichnet, dass** man die Verbindung der Formel I in einer heißen Lipid/Tensidlösung hochdruckhomogenisiert und anschließend abkühlt.

10. Verfahren zur Herstellung der Zubereitung gemäß einem oder mehreren der Ansprüche 4 bis 6, **dadurch gekennzeichnet, dass** man die Verbindung der Formel I mit bei Raumtemperatur flüssigen Lipiden hochdruckhomogenisiert.

11. Verfahren zur Herstellung der Zubereitung gemäß der Ansprüche 9 und 10, **dadurch gekennzeichnet, dass** in einem Gefäß ein Tensid und Wasser und in einem anderen Gefäß die Verbindung der Formel I und ein Lipid eingewogen werden, der Inhalt der beiden Gefäße auf eine Temperatur, die etwa 10 °C über dem Schmelzpunkt des genannten Lipids liegt, erwärmt wird, anschließend wird der Inhalt der beiden Gefäße vereinigt und das Gemisch unter Verwendung eines Hochdruckhomogenisators homogenisiert und abschließend abgekühlt, wobei das Lipid unter Ausbildung von Lipid-Nanopartikeln auskristallisiert.

12. Verfahren gemäß einem oder mehreren der Ansprüche 9 bis 11, **dadurch gekennzeichnet, dass** als Lipid Precirol, Compritol, Monosteol, Imwitor, Softisan, Phosphatidylethanolamin oder eine Mischung der Lipide und als Tensid Poloxamer eingesetzt wird.

13. Verwendung der Verbindung der Formel I gemäß einem oder mehreren der Ansprüche 1 bis 3 zur Herstellung eines Arzneimittels zur Behandlung der androgenetischen Alopezie, des Hirsutismus, der Seborrhö oder Akne.

14. Verwendung der Verbindung der Formel I gemäß einem oder mehreren der Ansprüche 1 bis 3 oder der Zubereitung gemäß einem oder mehreren der Ansprüche 4 bis 6 in der Kosmetik.

## Claims

1. Compound of the formula I 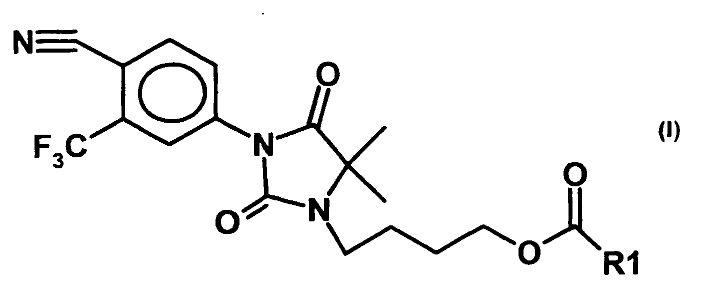 and/or a stereoisomeric form of the compound of the formula I
and/or a physiologically tolerated salt of the compound of the formula I, in which
R1 is -(C₅-C₁₇)-alkyl or -(C₅-C₁₇)-alkenyl.

2. Compound of the formula I according to Claim 1, **characterized in that**
R1 is -(C₁₁-C₁₅)-alkyl or-(C₁₁-C₁₅)-alkenyl.

3. Compound of the formula I according to Claim 1 or 2, **characterized in that** it is the compound of the formula II

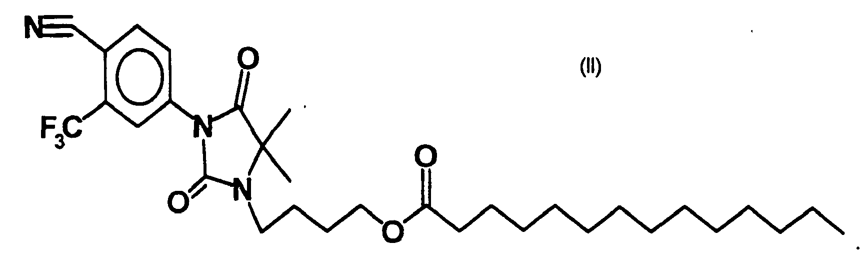

4. Pharmaceutical preparation comprising at least one lipid nanoparticle and at least one compound of the formula I according to Claim 1 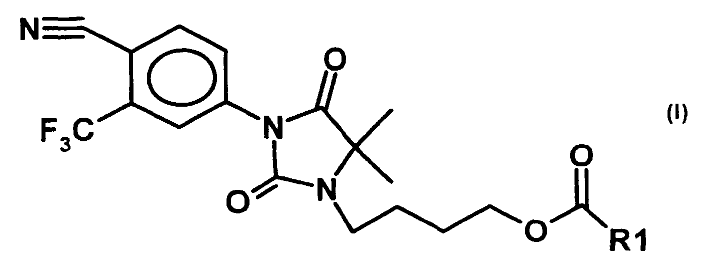 and/or one stereoisomeric form of the compound of the formula I
and/or one physiologically tolerated salt of the compound of the formula I, in which
R1 is -(C₅-C₁₇)-alkyl or -(C₅-C₁₇)-alkenyl.

5. Pharmaceutical preparation according to Claim 4, **characterized in that** it comprises a compound of the formula I in which
R1 is -(C₁₁-C₁₅)-alkyl or -(C₁₁-C₁₅)-alkenyl.

6. Pharmaceutical preparation according to Claim 4 or 5, **characterized in that** it comprises a compound of the formula II

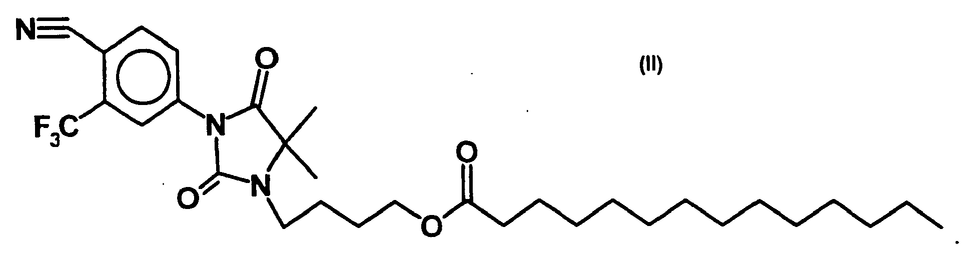

7. Process for preparing the compound of the formula I according to one or more of Claims 1 to 3, **characterized in that** it comprises
a) reacting a compound of the formula III 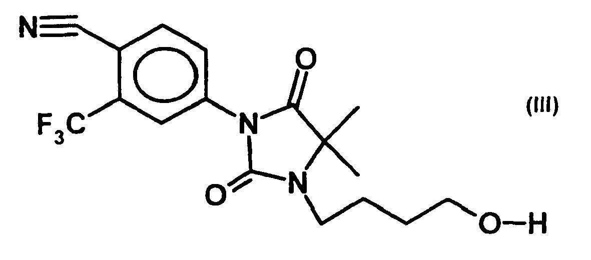 with an activated fatty acid of the formula IV 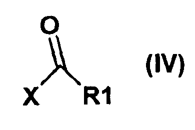 in which R1 is as defined in formula I, and X is a halogen radical, to give a compound of the formula I, or
b) fractionating a compound of the formula I which has been prepared by process a) and which, because of its chemical structure, occurs in enantiomeric forms, by salt formation with enantiopure acids or bases, chromatography on chiral stationary phases or derivatization using chiral compounds such as amino acids, separation of the diastereomers obtained in this way, and elimination of the chiral groups, into the pure enantiomers, or
c) either isolating the compound of the formula I which has been prepared by process a) in free form or, in the cases where acidic or basic groups are present, converting it into physiologically tolerated salts.

8. Medicament, **characterized by** an effective content of at least one compound of the formula I according to one or more of Claims 1 to 3 together with a pharmaceutically suitable and physiologically tolerated carrier, additive and/or other active ingredients and excipients.

9. Process for producing the pharmaceutical preparation according to one or more of Claims 4 to 6, **characterized in that** it comprises high-pressure homogenization of the compound of the formula I in a hot lipid/surfactant solution and subsequent cooling.

10. Process for producing the preparation according to one or more of Claims 4 to 6, **characterized in that** it comprises high-pressure homogenization of the compound of the formula I with lipids which are liquid at room temperature.

11. Process for producing the preparation according to Claims 9 and 10, **characterized in that** a surfactant and water are weighed into one vessel, and the compound of the formula I and a lipid are weighed into another vessel, the contents of the two vessels are heated to a temperature which is about 10°C above the melting point of said lipid, and then the contents of the two vessels are combined and the mixture is homogenized using a high-pressure homogenizer and finally cooled, whereupon the lipid crystallizes out to form lipid nanoparticles.

12. Process according to one or more of Claims 9 to 11, **characterized in that** Precirol, Compritol, Monosteol, Imwitor, Softisan, phosphatidylethanolamine or a mixture of the lipids is employed as lipid, and poloxamer is employed as surfactant.

13. Use of the compound of the formula I according to one or more of Claims 1 to 3 for producing a medicament for the treatment of androgenic alopecia, of hirsutism, of seborrhoea or acne.

14. Use of the compound of the formula I according to one or more of Claims 1 to 3 or of the preparation according to one or more of Claims 4 to 6 in cosmetics.

## Revendications

1. Composé de formule I 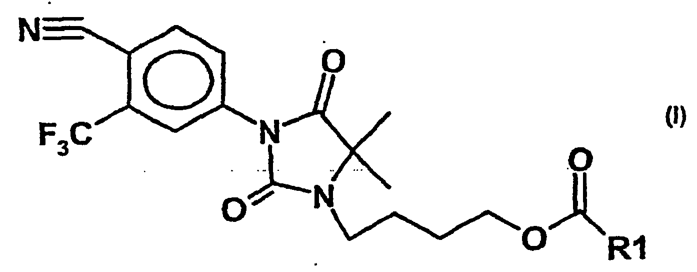 et/ou forme stéréoisomère du composé de formule I
et/ou sel physiologiquement acceptable du composé de formule I, formule dans laquelle R1 représente un groupe alkyle en C₅-C₁₇ ou alcényle en C₅-C₁₇.

2. Composé de formule I selon la revendication 1, **caractérisé en ce que** R1 représente un groupe alkyle en C₁₁-C₁₅ ou alcényle en C₁₁-C₁₅.

3. Composé de formule I selon la revendication 1 ou 2, **caractérisé en ce qu'**il est le composé de formule II

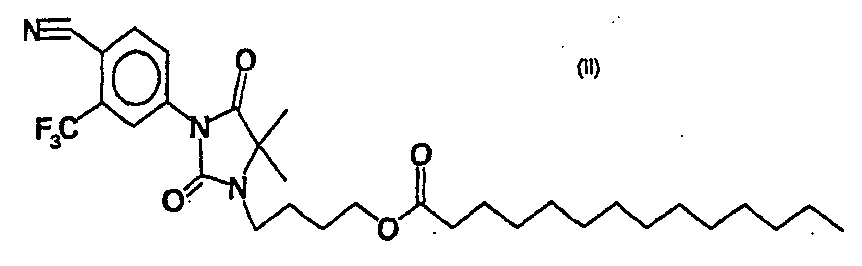

4. Préparation pharmaceutique contenant au moins une nanoparticule lipidique et au moins un composé de formule I selon la revendication 1, 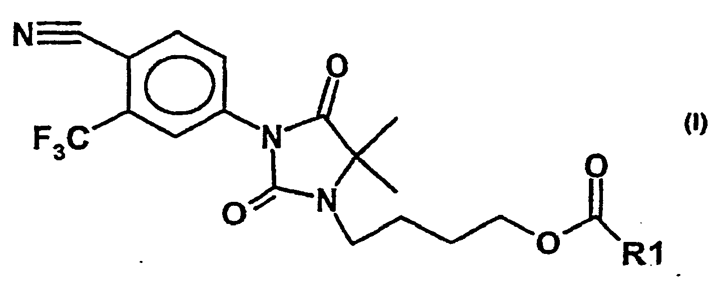 et/ou une forme stéréoisomère du composé de formule I et/ou un sel physiologiquement acceptable du composé de formule I, formule dans laquelle R1 représente un groupe alkyle en C₅-C₁₇ ou alcényle en C₅-C₁₇.

5. Préparation pharmaceutique selon la revendication 4, **caractérisée en ce qu'**elle contient un composé de formule I dans lequel R1 représente un groupe alkyle en C₁₁-C₁₅ ou alcényle en C₁₁-C₁₅.

6. Préparation pharmaceutique selon la revendication 4 ou 5 **caractérisée en ce qu'**elle contient un composé de formule II

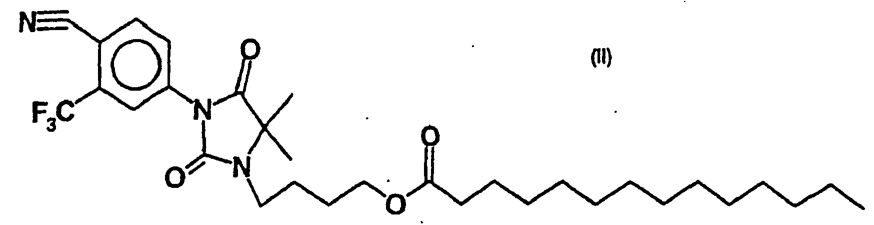

7. Procédé pour la préparation du composé de formule I selon une ou plusieurs des revendications 1 à 3,
**caractérisé en ce que**
a) on fait réagir un composé de formule III 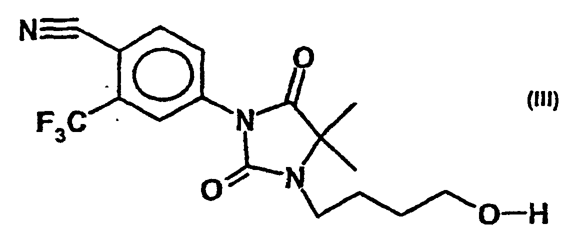 avec un acide gras activé de formule IV 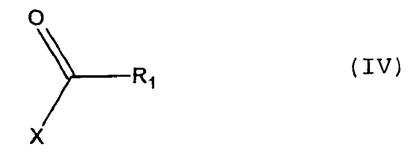 dans laquelle R1 est tel que défini dans la formule I et X est un atome d'halogène, pour obtenir un composé de formule I, ou
b) on sépare en les énantiomères purs un composé de formule I préparé selon le procédé a), qui, en raison de sa structure chimique, apparaît sous des formes énantiomères, par salification avec des acides ou des bases sous forme d'énantiomères purs, chromatographie sur des phases stationnaires chirales ou transformation en dérivés au moyen de composés chiraux tels que des aminoacides, séparation des diastéréoisomères ainsi obtenus, et élimination des groupes auxiliaires chiraux, ou
c) le composé de formule I préparé selon le procédé a) soit est isolé sous forme libre, soit, dans le cas de la présence de groupes acides ou basiques, converti en sels physiologiquement acceptables.

8. Médicament **caractérisé par** une teneur efficace en au moins un composé de formule I selon une ou plusieurs des revendications 1 à 3, conjointement avec un véhicule, un additif, pharmaceutiquement appropriés et physiologiquement acceptables et/ou d'autres adjuvants et principes actifs.

9. Procédé pour la production de la préparation pharmaceutique selon une ou plusieurs des revendications 4 à 6, **caractérisé en ce qu'**on homogénéise sous haute pression le composé de formule I dans une solution chaude de lipide/tensioactif et ensuite on le refroidit.

10. Procédé pour la production de la préparation selon une ou plusieurs des revendications 4 à 6, **caractérisé en ce qu'**on homogénéise sous haute pression le composé de formule I avec des lipides liquides à la température ambiante.

11. Procédé pour la production de la préparation selon une ou plusieurs des revendications 9 et 10, **caractérisé en ce qu'**on pèse dans un récipient un tensioactif et de l'eau et dans un autre récipient le composé de formule I et un lipide, on porte le contenu des deux récipients à une température qui est supérieure d'environ 10°C au point de fusion dudit lipide, on réunit ensuite le contenu des deux récipients et on homogénéise le mélange en utilisant un homogénéisateur à haute pression et ensuite on le refroidit, de sorte que le lipide cristallise avec formation de nanoparticules lipidiques.

12. Procédé selon une ou plusieurs des revendications 9 à 11, **caractérisé en ce qu'**on utilise comme lipide le précirol, le compritol, le monostéol, l'imwitor, le softisan, la phosphatidyléthanolamine ou un mélange des lipides et, comme tensioactif, le poloxamer.

13. Utilisation du composé de formule I selon une ou plusieurs des revendications 1 à 3, pour la fabrication d'un médicament destiné au traitement de l'alopécie androgénétique, de l'hirsutisme, de la séborrhée ou de l'acné.

14. Utilisation du composé de formule I selon une ou plusieurs des revendications 1 à 3 ou de la préparation selon une ou plusieurs des revendications 4 à 6, en cosmétique.
